**Europäisches Patentamt**

**European Patent Office**

**Office européen des brevets**

(11) Numéro de publication : **0 453 340 B1**

(12) **FASCICULE DE BREVET EUROPEEN**

(45) Date de publication du fascicule du brevet :
**19.01.94 Bulletin 94/03**

(51) Int. Cl.$^5$ : **B01J 29/06**, **C01B 33/34**

(21) Numéro de dépôt : **91400778.6**

(22) Date de dépôt : **22.03.91**

(54) **Procédé de synthèse d'un aluminosilicate cristallin enrichi en silice, ayant la structure de l'offretite.**

(30) Priorité : **23.03.90 FR 9003704**

(43) Date de publication de la demande :
**23.10.91 Bulletin 91/43**

(45) Mention de la délivrance du brevet :
**19.01.94 Bulletin 94/03**

(84) Etats contractants désignés :
**BE DE ES GB IT NL**

(56) Documents cités :
**EP-A- 0 214 042**
**EP-A- 0 251 892**
**FR-A- 2 575 741**
**GB-A- 1 061 847**
**US-A- 3 578 398**
**US-A- 3 591 488**
**US-A- 3 691 099**

(73) Titulaire : **SOCIETE NATIONALE ELF
AQUITAINE
Tour Elf, 2, Place de la Coupole, La Défense 6
F-92400 Courbevoie (FR)**

(72) Inventeur : **Fajula, François
6, rue du Jeu de Mail
F-34160 Theyran (FR)**
Inventeur : **Patarin, Joel
13, rue Eugène Delacroix
F-68093 Mulhouse (FR)**
Inventeur : **des Courieres, Thierry
152, boulevard Yves Farge
F-69007 Lyon (FR)**
Inventeur : **Fitoussi, Fredj
19, rue des Trois Pierres
F-69007 Lyon (FR)**

(74) Mandataire : **Boillot, Marc
SOCIETE NATIONALE ELF AQUITAINE
Division Propriété Industrielle Tour Elf
F-92078 Paris la Défense Cédex 45 (FR)**

## Description

La présente invention concerne la synthèse d'un aluminosilicate cristallin enrichi en silice, ayant la structure de l'offretite.

Identifiée pour la première fois par le Professeur GONNARD en 1890 dans les basaltes du Mont Simiouse, l'offretite ne fut définitivement identifiée et distinguée de l'erionite qu'en 1967 par BENNET et GARD (Nature 214 1005 (1967)).

L'offretite appartient à la famille de la chabazite et cristallise dans le système hexagonal. Sa structure est constituée d'un empilement de cages "cancrinites" reliées par des prismes hexagonaux. Les cages "cancrinites" ont des ouvertures à 6 côtés de 0,18 nm de diamètre. Ces empilements sont reliés entre eux par des cages "gmelinites". Ces cages ont des ouvertures à 8 côtés, accessibles à des molécules dont le diamètre critique est d'environ 0,5 nm. L'agencement dans l'espace de ces entités conduit à une structure poreuse caractérisée par des canaux de 0,64nm de diamètre, accessibles au travers d'ouvertures à 12 tétraèdres. L'offretite se classe ainsi parmi les zéolithes dont l'ouverture de pores est de taille moyenne.

L'erionite présente les mêmes empilements de cages cancrinites et de prismes, mais une rotation de 60° entre deux cages cancrinites successives provoque une obstruction périodique des canaux. Dans certains cas il y a intercroissance entre l'offretite et l'érionite, ce qui ajoute à la difficulté d'identification. La diffraction des rayons X sur poudre ne permet pas de distinguer ces deux types structuraux.

Il est cependant possible de distinguer ces deux structures par d'autres méthodes comme la diffraction électronique, la diffraction des rayons X sur monocristal ou bien la mesure de l'indice de contrainte (IC).

Selon cette dernière méthode, développée par FRILETTE (J.CATAL. 67,218,(1981)), on compare les vitesses relatives de craquage de n-hexane et du méthyl-3-pentane.

L'indice de contrainte (IC) est défini comme étant le rapport du logarithme de la fraction de n-hexane non converti sur le logarithme de la fraction de méthyl-3-pentane non converti. Le test s'effectue à pression atmosphérique sur un mélange équimoléculaire. On peut soit observer l'évolution de l'indice de contrainte en fonction du temps, soit fixer un temps donné, en général 20mn.

En l'absence de contrainte stérique l'indice de contrainte est faible (inférieur à 1). Des contraintes stériques liées à la structure (zéolithe à petits pores) ou au vieillissement (retrécissement des pores par cokage) conduisent à des valeurs élevées. Par exemple l'érionite, en raison de l'obstruction périodique des canaux, donne une valeur largement supérieure à 10.

Une des utilisations majeures des zéolithes est la catalyse acide. En effet les zéolithes sont des aluminosilicates composés d'enchaînements de tétraèdres $SiO_4$ et $AlO_4^-$. La neutralité électrique est assurée par des cations de compensation. En général ces cations sont des ions $K^+$ ou $Na^+$ échangeables par $NH_4^+$. Un traitement thermique permet de transformer $NH_4^+$ en $H^+$ et donc d'obtenir des solides acides.

Il est bien connu que pour chaque réaction catalysée par une zéolithe la fraction molaire aluminique m qui est définie comme le rapport atomique $\dfrac{Al}{(Al + Si)}$ joue un rôle fondamental.

Elle détermine directement la densité de sites acides. Une variation de la fraction aluminique entraîne une variation du nombre et de la force des sites acides. Cette variation peut être obtenue lors de la synthèse ou par un traitement post-synthèse.

Dans le cas d'un traitement post-synthèse tel qu'un traitement hydrothermique cette variation est accompagnée de la création d'une mésoporosité, ce qui est particulièrement important dans le cas des zéolithes à structure unidirectionnelle car cette mésoporosité permet de connecter les canaux de la zéolithe. Il existe donc un optimum de fraction aluminique fonction de la zéolithe et de la réaction considérée.

La ZSM-5, zéolithe découverte par Mobil et dont la structure de type MFI est décrite dans l'"Atlas of zeolite structure types" de Meier et Olson (édition de 1987 Butterworth) est obtenue par synthèse directe avec une fraction aluminique faible, inférieure à 0,1.

L'offretite dont la structure est décrite dans le même atlas résulte de la cristallisation hydrothermale d'un gel aluminosilicate pris dans le système comportant une source de silicium, une source d'aluminium, un alcalin qui est en général du potassium et un structurant qui est en général un ion ammonium quaternaire tel que l'ion tétraméthylammonium. A l'issue de la synthèse, la fraction molaire aluminique est d'environ 0,2.

Dans le cas de l'offretite, la synthèse directe ne permet pas de varier la fraction aluminique de façon significative. La seule voie qui s'ouvre alors pour varier ce rapport est celle de la modification de la charpente déjà formée. Cette voie n'est pas sans danger, la structure cristalline de l'offretite étant très facilement détruite par ces traitements. Ainsi, la desalumination de l'offretite par traitement acide semblait longtemps impossible, la charpente étant détruite par ces traitements. (F. HERNANDEZ, R.IBARRA F.FAJULA, F.FIGUERAS, Acta Phys.Chem.31,81 (1985)).

Un procédé de desalumination de l'offretite a été mis au point récemment par l'Institut Français du Pétrole.

Ce procédé, décrit dans la demande de brevet européen 190949 nécessite de nombreuses étapes de calcination, d'échange cationique, de traitement à la vapeur et d'attaque acide.

La multiplication de ces étapes élémentaires rend aléatoire et en tout état de cause extrêmement chère une industrialisation de ce procédé.

Nous avons trouvé maintenant un procédé simplifié qui permet d'obtenir en peu d'étapes un aluminosilicate cristallin, ayant la structure de l'offretite, de fraction aluminique faible.

Cet aluminosilicate présente une bonne sélectivité dans les réactions de catalyse acide et se caractérise par sa bonne stabilité.

Notre procédé de synthèse d'un aluminosilicate cristallin enrichi en silice, ayant la structure de l'offretite comporte les étapes suivantes :

- préparation d'un gel de nucléation A contenant des sources de silicium, d'aluminium, d'ions alcalins (M), un structurant organique (Z) et l'eau
- repos de ce mélange
- préparation d'un gel de croissance B contenant une source de silicium, d'aluminium, d'ions alcalins et de l'eau
- ajout d'au moins 2 % poids de gel A vieilli au gel B frais
- chauffage du gel A-B sous agitation pour obtenir sa cristallisation, puis séparation, lavage et séchage des cristaux
- calcination à une température suffisante pour éliminer le structurant organique
- échange des ions alcalins au proton
- traitement hydrothermique
- traitement par un acide minéral fort en milieu aqueux, puis séparation, lavage et conditionnement des cristaux. Selon le type de procédé catalytique, ce conditionnement peut être une calcination, une atomisation ou directement une extrusion en présence d'un liant.

Une variante du procédé consiste à éliminer le structurant en même temps que les ions potassium sans passer par une étape de calcination préalable.

Les gels A et B renferment les mêmes sources de silice, d'alumine et de métal alcalin mais seul le gel A renferme l'agent structurant. Les stoechiométries peuvent être modifiées à condition de rester dans le domaine de la cristallisation de l'offretite pure, c'est-à-dire de rester dans des conditions pour lesquelles l'indice de contrainte de l'offretite obtenue est inférieur à 2.

Pour obtenir une offretite pure, d'indice de contrainte inférieur à 2, les rapports molaires des réactifs dans les gels A et B doivent rester entre les limites suivantes :

$$SiO_2/Al_2O_3 = 5\text{-}40$$
$$SiO_2/\text{ion alcalin} = 1\text{-}2,6$$
$$\text{Structurant/ion alcalin} = 0\text{-}0,3$$
$$H_2O/\text{ion alcalin} = 27\text{-}50$$

Comme ion alcalin on utilise en général du potassium et/ou du sodium. On utilise de préférence du potassium, sous forme de KOH.

Parmi les structurants organiques, les tétraalkylammonium sont plus couramment utilisés et de préférence le tétraméthylammonium.

La source de silicium peut être choisie parmi les silicates, les silices solides, les silices colloidales, les gels ou xerogels de silice ou la diatomite.

La source d'aluminium est en général un sel d'aluminium, comme l'aluminate de sodium ou de potassium ou l'oxyde ou l'hydroxyde d'aluminium.

On peut avantageusement utiliser les aluminosilicates cristallins, naturels ou synthétiques, comme le kaolin, la metakaolinite, la perlite ou les zéolithes. Dans ce cas le rapport silicium/aluminium peut être ajusté par une deuxième source de silicium ou aluminium. Tout ou partie des eaux-mères issues d'une première synthèse peut également être utilisé dans la préparation du gel B

Une stoechiométrie type est la suivante :

```
Gel A  6K₂O, 3(TMA)₂O, 1Al₂O₃, 16 SiO₂, 300H₂O
Gel B  6K₂O,           1Al₂O₃, 16 SiO₂, 300H₂O
```

Le gel de nucléation A, constitué de sources de silicium, d'aluminium, d'ions alcalins, de structurant organique et d'eau, est laissé au repos pendant environ 12 à 300 heures à une température comprise entre 10 et 100°C mais en général proche de la température ambiante. 2 à 50 % et de préférence 5 à 20 % poids de ce gel de nucléation A vieilli est ajouté, au gel de croissance B fraîchement préparé.

Le mélange A-B cristallise, sous pression autogène, sous agitation ou non, à une température comprise entre 20 à 200°C et de préférence entre 100 et 150°C pendant 2 à 50 et de préférence entre 8 et 20 heures.

Les cristaux de l'offretite sont séparés en général par filtration, puis lavés et séchés.

L'étape de calcination a pour but d'éliminer le structurant organique. Elle nécessite en général un chauffage pendant 15 minutes à 5 heures à une température supérieure à 500°C.

Pour l'échange des ions alcalins on utilise en général une solution d'un sel d'ammonium, comme l'acétate d'ammonium, le sulfate d'ammonium ou le nitrate d'ammonium. En fonction du type d'appareillage et de la température il est parfois nécessaire d'effectuer plusieurs échanges successifs. Classiquement le taux d'échange est d'environ 70% ce qui correspond à une teneur résiduelle en ion alcalin de 2 à 3% poids dans le cas du potassium.

Le traitement hydrothermique se déroule à une température comprise entre 500 et 850°C sous 100% de vapeur d'eau pendant une durée inférieure à 5 heures. Il est possible d'effectuer le traitement hydrothermique sur produit non séché, en atmosphère statique. L'humidité dans ce cas est apportée par le produit lui-même (Self-steaming).

Pour le traitement par un acide minéral fort on utilise en général un acide minéral tel que l'acide nitrique, l'acide sulfurique ou l'acide chlorhydrique. En sélectionnant judicieusement la concentration et la durée de traitement ainsi que la température on peut éliminer les débris aluminiques sans affecter la cristallinité. En général ce traitement est effectués à reflux pendant une durée inférieure à 5 heures.

L'aluminosilicate obtenu a un indice de contrainte inférieur à 2, la fraction molaire aluminique est inférieure à 0,15. Ces valeurs montrent que l'aluminosilicate enrichi en silice obtenu, ayant la structure de l'offretite, a conservé sa structure cristalline et sa porosité.

L'offretite obtenue peut être utilisé comme catalyseur pure, sous forme, par exemple, de poudre ou de grains. Il peut être avantageux d'ajouter un liant inerte, comme par exemple l'alumine, la silice, les silice-alumines ou les argiles.

Il est également possible d'utiliser cette offretite en combinaison avec d'autres catalyseurs notamment d'autres zéolithes, ou bien échangée aux terres rares.

Les catalyseurs obtenus sont utilisables pour des réactions de transformation des hydrocarbures. Nous pouvons mentionner l'isomérisation des alkanes et les alkylaromatiques comme le xylène, la disproportionation du toluène, l'alkylation des noyaux aromatiques dans la synthèse de l'éthylbenzène et l'aromatisation de l'octane en styrène. Ils conviennent également pour l'oligomérisation des oléfines à chaîne courte pour la synthèse des lubrifiants et le déparaffinage des lubrifiants ou d'autres fractions pétrolières renfermant des paraffines. Ils peuvent être utilisés pour l'hydratation des oléfines. Comme catalyseur FCC ils augmentent le rendement en GPL et l'indice d'octane de l'essence obtenu.

Ils sont utiles comme catalyseurs dans la synthèse des essences à partir du méthanol ou par le procédé Fischer-Tropsch.

L'offretite obtenue est également utile comme tamis moléculaire, par exemple pour la séparation des isomères du xylène et de l'éthylbenzène et le séchage des composés organiques.

Les exemples suivants illustrent l'invention sans toutefois la limiter.

## EXEMPLE 1

On prépare un gel A de nucléation de composition molaire $6K_2O$, $3(TMA)_2 O$, $1Al_2O_3$, $16 SiO_2$, $300H_2O$ à partir de potasse en pastilles à 85%, de métakaolin, de silice et d'un sel d'ions tétraméthylammonium.

Le mélange est vieilli sous faible agitation à la température ambiante pendant 3 jours.

Un second gel B de croissance ayant la composition molaire $6K_2O$, $1Al_2O_3$, $16 SiO_2$, $300H_2O$ est obtenu à partir des mêmes composants que le gel A mais en l'absence d'agent structurant.

Un mélange à 10% du gel A et 90% poids du gel B est transvasé dans un autoclave agité où il est maintenu à 150°C pendant 12 heures.

Les cristaux d'offretite obtenus se présentent sous la forme de prismes hexagonaux aux formes régulières de 0,5 à 1 $\mu$n de long environ, d'un rapport longueur sur diamètre de 2 à 3.

L'examen de ces cristaux par diffraction électronique en champ sombre révèle une offretite parfaite alors que sur un échantillon classique d'offretite on détecte la présence de défauts structuraux de type erionite.

L'activation de cette zéolite a été réalisée en utilisant une procédure banale :

- lavage pour éliminer les eaux-mères
- calcination sous air à 550°C pour éliminer l'agent structurant
- échange au sulfate d'ammonium pour éliminer la majeure partie du potassium
- traitement hydrothermique à 500 ou 800°C pour provoquer la désalumination plus ou moins poussée du réseau

- lavage à l'acide sulfurique 1N à reflux pour éliminer les débris amorphes

1a) L'échantillon traité à 500°C a une fonction molaire aluminique de 0,11. Sa cristallinité mesurée aux rayons X par la méthode de poudres est de 100% par rapport à l'offretite calcinée. L'indice de contrainte IC, déterminé à 350°C au bout de 20 minutes sur un mélange équimoléculaire de n-hexane et de méthyl-3-pentane est de 1,0.

1b) L'échantillon traité à 800°C a une fraction molaire aluminique de 0,06. Sa cristallinité mesurée aux rayons X par la méthode de poudres est de 95% par rapport à l'offretite calcinée. L'indice de contrainte IC, déterminé à 350°C au bout de 20 minutes sur un mélange équimoléculaire de n-hexane et de méthyl-3-pentane est de 1,0.

Les propriétés catalytiques de ces deux offretites sont décrites dans les exemple ci-dessous.

## EXEMPLE 2

Isomérisation des coupes C5-C6.

Un catalyseur d'isomérisation des paraffines linéaires en C5-C6 a été préparé en mélangeant mécaniquement une offretite désaluminée ayant une fraction molaire d'aluminium m=Al/Al+Si de 0.11 (Si/Al=8) avec un catalyseur d'hydrogénation Pt sur alumine (teneur finale en platine 0,5%).

Ce catalyseur a été utilisé pour isomériser du n-pentane à 220°C, 1 atmosphère de pression totale, un rapport Hydrogène sur Hydrocarbure de 70 et une vitesse spatiale (poids de pentane/poids de zéolithes * temps en heures) de 0,2 h-1.

La conversion obtenue a été de 30% avec une sélectivité de 100% en isopentane. Dans l'isomérisation du n-hexane, avec les mêmes conditions opératoires, la conversion obtenue a été de 45% avec une sélectivité de 90% en isomères et de 10% en produits de craquage.

Evalué dans des conditions opératoires identiques, un catalyseur d'isomérisation commercial à base de mordénite désaluminée a donné les résultats suivants :

- Réaction du n-pentane : 16% de conversion avec une sélectivité de 80% en isopentane et 20% en produits de craquage,
- Réaction du n-hexane : 30% de conversion avec une sélectivité de 70% en isomères et 30% en produits de craquage.

Pour les deux réactions, le catalyseur à base d'offretite désaluminée (m=0,11), s'est donc avéré plus actif et plus sélectif que le catalyseur de référence à base de mordénite.

## EXEMPLE 3

Alkylation du benzène par l'éthanol.

Une offretite dont le rapport m=0,11 a été testée dans l'alkylation du benzène par l'éthanol à 250°C, à 1 atmosphère de pression, un rapport benzène/éthanol de 7 et une vitesse spatiale de 4 h-1. Une vitesse de conversion du benzène de 3,56 mmoles/g*h a été obtenue avec une sélectivité de 92% en éthylbenzène.

Dans les mêmes conditions de test, une offretite non désaluminée (m=0,28, Si/Al=2.5) donne une vitesse de 0,3 mmole/g*h et une sélectivité de 30%.

## EXEMPLE 4

Conversion du méthanol en oléfines.

Une offretite désaluminée de rapport m=0,062 (Si/Al=15) a été utilisée pour convertir du méthanol en oléfines à 370°C, 1 atmosphère de pression, un débit de méthanol de 20 ml/h, un débit d'azote de 9 1/h. La masse de zéolithe était de 1,5g. Après trois heures de réaction la conversion du méthanol était de 100% et la distribution des produits la suivante :

| Méthane | 30,4 % |
|---|---|
| Ethane + Ethylène | 40,7 % |
| Propane | 12,2 % |
| Propylène | 11,2 % |
| Butane | 1,6 % |
| Butènes | 3,9 % |

Une offretite non désaluminée testée dans les mêmes conditions ne conduit, après 30 minutes de réaction, qu'à 29% de diméthyléther et est totalement inactive après trois heures de réaction.

EXEMPLE 5

Conversion d'une charge mixte éthylbenzène-xylènes.

Une offretite de rapport m=0,062 est mélangée à un catalyseur d'hydrogénation platine sur alumine de façon à obtenir un catalyseur bifonctionnel Pt/zéolithe à 0,3% de Pt.

Ce catalyseur est utilisé pour transformer une charge contenant 25% d'éthylbenzène et 75% de o-xylène à 380°C, sous 10 atmosphères de pression, avec un rapport hydrogène/hydrocarbure de 4 et une vitesse spatiale de 40h-1. Les conversions obtenues sont de 82% sur l'ortho-xylène et de 55% sur l'éthylbenzène avec un rendement en xylènes de 92%.

Dans les mêmes conditions de réaction, un catalyseur de référence à base de mordénite désaluminée conduit à des conversions de 70% et 42% pour l'o-xylène et l'éthylbenzène respectivement avec un rendement en xylènes de 51%.

EXEMPLE 6

Nous avons mesuré l'efficacité de l'offretite selon l'invention comme catalyseur de FCC par une méthode de laboratoire selon la norme ASTM D 3907-87.

La charge hydrocarboné traitée est une charge typique prélevée en raffinerie, dont nous résumons les caractéristiques =

$d_4^{15}$ = 0,922
point d'aniline = 82,1°C
Viscosité à 100°C=6,08.10$^{-6}$m$^2$/s
Soufre = 2,68%
Azote = 800ppm
Carbon Conradson = 0,44%
Distillation selon ASTM D-1160

| | |
|---|---|
| 5% | 324°C |
| 10% | 361°C |
| 50% | 431°C |
| 90% | 502°C |
| 95% | 519°C |

Nous avons utilisé trois catalyseurs, A, B et C =

A= catalyseur industriel de la Société CROSFIELD, soumis à un traitement à la vapeur, à 750°C, pendant 17heures sous 100% de vapeur d'eau.

B=95% de catalyseur A + 5% d'une offretite fraîche de fraction aluminique = 0,11

C=95% de catalyseur A + 5% de ZSM-5 fraîche. Cette ZSM-5 est identique à celle utilisée industriellement dans les craqueurs catalytiques.

Conditions opératoires = 6 grammes de catalyseur, température = 530°C, rapport catalyseur/charge=6, temps d'injection = 20 secondes, correspondant à un poids/poids/heure de 30g/g.h.

Les résultats sont résumés dans le tableau 1.

EP 0 453 340 B1

## TABLEAU I

|  | A | B | C |  |
|---|---|---|---|---|
|  | (comparatif) |  | (comparatif) |  |
| $H_2$ | 0,05 | 0,07 | 0,08 |  |
| $C_1$ | 0,78 | 1,09 | 1,05 |  |
| $C_2$ | 0,55 | 0,77 | 0,93 |  |
| $C_2=$ | 1,56 | 2,57 | 3,94 |  |
| $C_3$ | 2,34 | 5,01 | 5,30 |  |
| $C_3=$ | 8,05 | 8,52 | 8,36 |  |
| $iC_4$ | 7,02 | 10,89 | 8,44 |  |
| $nC_4$ | 1,45 | 2,64 | 2,37 |  |
| $C_4=$ | 7,34 | 6,10 | 5,49 |  |
| gas $(H_2+C_1+C_2)$ | 2,94 | 4,50 | 6,00 |  |
| GPL $(C_3+C_4)$ | 26,20 | 33,16 | 29,96 |  |
| Essence $(C_5-215)$ | 41,58 | 36,32 | 34,58 |  |
| Gazole $(215-350°C)$ | 15,32 | 14,01 | 15,94 |  |
| Résidu $(350°C^+)$ | 9,56 | 6,90 | 8,58 |  |
| Coke | 4,40 | 5,11 | 4,94 |  |

L'offretite est plus sélective que la ZSM5. Elle permet d'augmenter la quantité de GPL sans trop perdre en rendement essence.

De plus sa meilleure sélectivité en isobutane laisse présager un gain supérieur en indice d'octane et tout particulièrement en indice d'octane moteur.

EXEMPLE 7 (comparatif)

Un mélange de composition stoechiométrique :
$$4,46 \ K_2O; \ 2,56 \ TMA_2O; \ Al_2O_3; \ 15,3 \ SiO_2; \ 280 H_2O$$
est préparé en dissolvant 0,73g de métakaolin dans une solution contenant 15 ml d'eau, 1,65g de potasse en pastilles (KOH) et 3,05g de tétraméthylammonium hydroxyde pentahydrate (TMAOH, $5H_2O$). Après avoir mélangé ces ingrédients on rajoute 2,63g de silice (gel de silice en grains).

L'ensemble est cristallisé à 110°C pendant 72 heures pour produire une offretite, bien cristallisée d'après son diffractogramme des rayons X, et dont la composition chimique est :
$$0,79 \ K_2O; \ 0,20 \ TMA_2O; \ Al_2O_3; \ 7,1 \ SiO_2; \ 7,2 H_2O.$$
Ce solide est transformé en une zéolithe échangée à l'ammonium par i) calcination à 500°C sous air pendant 4heures et ii) échange à reflux dans une solution 1N de $NH_4 NO_3$ pendant 10 heures avec un rapport liqui-

7

de/solide de 100/1. Le solide échangé a gardé sa cristallinité, évaluée par diffraction des rayons X, et sa teneur en ions alcalins est de 1,3 % en poids.

Ce solide a été désaluminé par traitement hydrothermique en atmosphère confinée (Self-steaming) à une température de 650°C pendant 2 heures.

Après désalumination sa cristallinité évaluée par diffraction des rayons X ne correspond qu'à 30 % de la cristallinité du solide initial et sa porosité n'est pas accessible à des molécules aussi petites que l'azote car son volume poreux mesuré par les techniques bien connues dans l'art est inférieur à 0,01 ml adsorbé par gramme de zéolithe.

L'attaque acide conduite sur le solide désaluminé pour en libérer la porosité a été réalisée dans les conditions suivantes :

- 50 ml d'HCl 0,5 N par gramme de zéolithe
- température ambiante
- 4 heures de réaction

Malgré des conditions douces, le solide après attaque acide, est entièrement amorphe.

Cet exemple montre que l'offretite désaluminée obtenue par une synthèse classique suivi d'une désalumination simple, perd sa cristallinité et sa porosité.

## Revendications

**1.** Procédé de synthèse d'offretite désaluminée, caractérisé en ce qu'il comporte les étapes suivantes :
- préparation d'un gel de nucléation A contenant des sources de silicium, d'aluminium, d'ions alcalins (M), un structurant organique (Z) et l'eau,
- repos de ce mélange
- préparation d'un gel de croissance B contenant une source de silicium, d'aluminium, d'ions alcalins et de l'eau mais ne contenant pas de structurant organique, les rapports molaires dans les gels A et B étant compris entre les limites suivantes :
  $SiO_2/Al_2O_3 = 5\text{-}40$
  $SiO_2$/ion alcalin = 1-2,6
  structurant/ion alcalin = 0-0,3
  $H_2O$/ion alcalin = 27-50
- ajout d'au moins 2 % poids de gel A vieilli au gel B frais
- chauffage du gel A-B sous agitation pour obtenir sa cristallisation, puis séparation, lavage et séchage des cristaux
- calcination à une température suffisante pour éliminer le structurant organique
- échange des ions alcalins au proton
- traitement hydrothermique à une température comprise entre 500 et 850°C sous 100 % de vapeur d'eau pendant une durée inférieure à 5 heures
- traitement par un acide minéral fort en milieu aqueux, puis séparation, lavage et conditionnement des cristaux.

**2.** Procédé selon la revendication 1 caractérisé en ce que l'ion alcalin est du potassium et/ou du sodium, de préférence du potassium sous forme de KOH.

**3.** Procédé selon les revendications 1 ou 2 caractérisé en ce que le structurant organique est un tétraalkylammonium et de préférence le tétraméthylammonium.

**4.** Procédé selon l'une des revendications 1 à 3 caractérisé en ce que la source de silicium est choisie parmi les silicates, les silices solides, les silices colloïdales, les gels ou xerogels de silice ou la diatomite.

**5.** Procédé selon l'une des revendications 1 à 4 caractérisé en ce que la source d'aluminium est un sel d'aluminium, comme l'aluminate de sodium ou de potassium, ou l'oxyde ou l'hydroxyde d'aluminium ou bien un aluminosilicate cristallin, naturel ou synthétique.

**6.** Procédé selon l'une des revendications 1 à 5 caractérisé en ce que le gel de nucléation A est laissé au repos pendant 12 à 300 heures à une température comprise entre 10 et 100°C, en général à la température ambiante.

**7.** Procédé selon l'une des revendications 1 à 6 caractérisé en ce qu'on ajoute 2 à 50 % et de préférence 5 à 20 % poids de gel A vieilli au gel B frais.

8. Procédé selon l'une des revendications 1 à 7 caractérisé en ce que le mélange A - B cristallise, sous pression autogène, à une température comprise entre 20 et 200°C et de préférence entre 100 et 150°C pendant 2 à 50 et de préférence entre 8 et 20 heures, puis les cristaux sont séparés, lavés et séchés.

9. Procédé selon l'une des revendications 1 à 8 caractérisé en ce que le structurant organique est éliminé par chauffage pendant 15 minutes à 5 heures à une température supérieure à 500°C.

10. Procédé selon l'une des revendications 1 à 9 caractérisé en ce que les ions alcalins sont échangés par une solution d'un sel d'ammonium comme l'acétate d'ammonium, le sulfate d'ammonium ou le nitrate d'ammonium.

11. Procédé selon la revendication 1 caractérisé en ce que la vapeur d'eau du traitement hydrothermique provient du produit non-séché.

12. Procédé selon l'une des revendications 1 à 11 caractérisé en ce que l'acide minéral fort utilisé pour le traitement acide est l'acide nitrique, l'acide sulfurique ou l'acide chlorhydrique.

13. Procédé selon l'une des revendications 1 à 12 caractérisé en ce que le traitement acide est effectué par chauffage à reflux pendant une durée inférieure à 5 heures.

**Patentansprüche**

1. Verfahren zur Synthese von entaluminiertem Offretit, dadurch gekennzeichnet, daß es folgende Stufen umfaßt:
   - Herstellung eines keimbildenden Gels A enthaltend Silizium-, Aluminium- und alkalische Ionenquellen (M), ein organisches Strukturierungsmittel (Z) und Wasser,
   - Ruhenlassen dieses Gemisches
   - Herstellung eines Wachstumsgels B enthaltend eine Silizium-, Aluminium-, alkalische Ionenquelle und Wasser, jedoch kein organisches Strukturierungsmittel, wobei die Molverhältnisse in den Gelen A und B in den folgenden Grenzwerten liegen:
     $SiO_2/Al_2O_3 = 5\text{-}40$
     $SiO_2/\text{alkalisches Ion} = 1\text{-}2,6$
     $\text{Strukturierungsmittel/alkalisches Ion} = 0\text{-}0,3$
     $H_2O/\text{alkalisches Ion} = 27\text{-}50$
   - Zugabe von wenigstens 2 Gew.-% des gealterten Gels A zum frischen Gel B
   - Erhitzen von Gel A-B unter Rühren, um seine Kristallisation zu erreichen, anschließend Trennen, Reinigen und Trocknen der Kristalle
   - Kalzinierung bei einer Temperatur, die ausreichend ist, um das organische Strukturierungsmittel zu entfernen
   - Austausch der alkalischen Ionen durch Protonen
   - hydrothermische Behandlung bei einer Temperatur zwischen 500 und 850°C unter 100%igem Wasserdampf während einer Dauer von unter 5 Stunden
   - Behandlung durch eine starke Mineralsäure in einem wässerigen Medium, anschließend Trennen, Reinigen und Aufbereiten der Kristalle.

2. Verfahren nach Anspruch 1, dadurch gekennzeichnet, daß das alkalische Ion Kalium und/oder Natrium ist, vorzugsweise Kalium in Form von KOH.

3. Verfahren nach den Ansprüchen 1 oder 2, dadurch gekennzeichnet, daß das organische Strukturierungsmittel ein Tetraalkylammonium ist, vorzugsweise Tetramethylammonium.

4. Verfahren nach einem der Ansprüche 1 bis 3, dadurch gekennzeichnet, daß die Siliziumquelle ausgewählt ist unter den Silikaten, festen Siliziumdioxiden, Kolloidsiliziumdioxiden, Siliziumdioxidgelen oder -xerogelen oder Diatomit.

5. Verfahren nach einem der Ansprüche 1 bis 4, dadurch gekennzeichnet, daß die Aluminiumquelle ein Aluminiumsalz ist, wie ein Natrium- oder Kaliumaluminat, oder ein Aluminiumoxid oder -hydroxid, oder ein natürliches oder synthetisches kristallines Alumosilikat.

6. Verfahren nach einem der Ansprüche 1 bis 5, dadurch gekennzeichnet, daß man das keimbildende Gel A während 12 bis 300 Stunden bei einer Temperatur zwischen 10 und 100°C, im allgemeinen bei der Umgebungstemperatur, ruhen läßt.

7. Verfahren nach einem der Ansprüche 1 bis 6, dadurch gekennzeichnet, daß man dem frischen Gel B 2 bis 50 Gew.-%, vorzugsweise 5 bis 20 Gew.-%, des gealterten Gels A zugibt.

8. Verfahren nach einem der Ansprüche 1 bis 7, dadurch gekennzeichnet, daß das Gemisch A - B unter Eigendruck bei einer Temperatur zwischen 20 und 200°C, vorzugsweise zwischen 100 und 150°C, während 2 bis 50, vorzugsweise zwischen 8 und 20 Stunden, kristallisiert wird, und anschließend die Kristalle getrennt, gereinigt und getrocknet werden.

9. Verfahren nach einem der Ansprüche 1 bis 8, dadurch gekennzeichnet, daß das organische Strukturierungsmittel durch Erhitzung während 15 Minuten bis 5 Stunden bei einer Temperatur höher als 500°C entfernt wird.

10. Verfahren nach einem der Ansprüche 1 bis 9, dadurch gekennzeichnet, daß die alkalischen Ionen durch eine Ammoniumsalzlösung wie Ammoniumazetat, Ammoniumsulfat oder Ammoniumnitrat ausgetauscht werden.

11. Verfahren nach Anspruch 1, dadurch gekennzeichnet, daß der Wasserdampf bei der hydrothermischen Behandlung von einem nichtgetrockneten Produkt stammt.

12. Verfahren nach einem der Ansprüche 1 bis 11, dadurch gekennzeichnet, daß die zur Säurebehandlung verwendete starke Mineralsäure Salpetersäure, Schwefelsäure oder Salzsäure ist.

13. Verfahren nach einem der Ansprüche 1 bis 12, dadurch gekennzeichnet, daß die Säurebehandlung durch Rücklauferhitzung während einer Zeitdauer von unter 5 Stunden durchgeführt wird.

## Claims

1. Process for synthesising dealuminised offretite, characterised in that it comprises the following stages:
   - preparing a nucleation gel A containing sources of silicon, aluminum, alkaline ions (M), an organic structuring agent (Z) and water;
   - resting this mixture;
   - preparing a growth gel B containing a source of silicon, aluminium, alkaline ions and water, but not containing any organic structuring agent, the molar ratios in the gels A and B being between the following limits:
        $SiO_2/Al_2O_3$ = 5-40
        $SiO_2$/alkaline ion = 1-2.6
        structuring agent/alkaline ion = 0-0.3
        $H_2O$/alkaline ion = 27-50
   - adding at least 2 weight % of the aged gel A to the fresh gel B;
   - heating the gel A-B under agitation in order to bring about its crystallisation, then separating, washing and drying the crystals;
   - calcining at a temperature which is sufficient to eliminate the organic structuring agent;
   - exchanging the alkaline ions on the proton;
   - hydrothermic treatment at a temperature of between 500 and 850°C at 100 % water steam for a period of less than 5 hours;
   - treatment with a strong mineral acid in an aqueous medium, then separating, washing and conditioning the crystals.

2. Process according to Claim 1, characterised in that the alkaline ion is potassium and/or sodium, preferably potassium in the form of KOH.

3. Process according to either of Claims 1 and 2, characterised in that the organic structuring agent is a tetraalkylammonium and preferably tetramethylammonium.

4. Process according to any one of Claims 1 to 3, characterised in that the silicon source is selected from among silicates, solid silicas, colloidal silicas, silica gels or xerogels or diatomite.

5. Process according to any one of Claims 1 to 4, characterised in that the aluminium source is an aluminum salt, such as sodium or potassium aluminate, or aluminum hydroxide or oxide, or even a natural or synthetic crystalline aluminosilicate.

6. Process according to any one of Claims 1 to 5, characterised in that the nucleation gel A is left to rest for 12 to 300 hours at a temperature of between 10 and 100°C, in general at ambient temperature.

7. Process according to any one of Claims 1 to 6, characterised in that 2 to 50% and preferably 5 to 20% by weight of the aged gel A is added to the fresh gel B.

8. Process according to any one of Claims 1 to 7, characterised in that the mixture A-B crystallises, at autogenous pressure, at a temperature of between 20 and 200°C and preferably between 100 and 150°C for from 2 to 50 and preferably from 8 to 20 hours, and the crystals are then separated, washed and dried.

9. Process according to any one of Claims 1 to 8, characterised in that the organic structuring agent is eliminated by heating for between 15 minutes and 5 hours at a temperature which is greater than 500°C.

10. Process according to any one of Claims 1 to 9, characterised in that the alkaline ions are exchanged for an ammonium salt solution such as ammonium acetate, ammonium sulphate or ammonium nitrate.

11. Process according to Claim 1, characterised in that the water steam from the hydrothermic treatment originates from the undried product.

12. Process according to any one of Claims 1 to 11, characterised in that the strong mineral acid used for the acid treatment is nitric acid, sulphuric acid or hydrochloric acid.

13. Process according to any one of Claims 1 to 12, characterised in that the acid treatment is performed by refluxing for a period of less than 5 hours.